# EUROPEAN PATENT SPECIFICATION

(11) **EP 1 143 251 B1**
(45) Date of publication and mention of the grant of the patent: **08.09.2004**
(21) Application number: 99900171.2
(22) Date of filing: 12.01.1999
(51) Int. Cl.: G01N 33/543

(54) **METHOD AND BIOSENSOR FOR DETECTING ANTIGEN**
VERFAHREN UND BIOSENSOR ZUM NACHWEIS VON ANTIGENEN
PROCEDE ET BIOCAPTEUR DE DETECTION D'ANTIGENE

(43) Date of publication of application: 10.10.2001
(73) Proprietor: EBARA CORPORATION, Ohta-ku, Tokyo 144-8510 (JP); Karube, Isao, Kawasaki-shi, Kanagawa 216-0002 (JP)
(72) Inventor: KARUBE, Isao, Kawasaki-shi Kanagawa 216-0002 (JP); NOMURA, Yoko, Tokyo 207-0002 (JP)
(74) Representative: HOFFMANN - EITLE
(86) International application number: PCT/JP1999/000066
(87) International publication number: WO 2000/042433

(56) References cited:
- WO-A-90/11525
- JP-A- 1 109 262
- JP-A- 4 501 605
- JP-A- 10 267 834
- JP-A- 63 014 691
- NAKAMURA R ET AL: "A plasma polymerized film for surface plasmon resonance immunosensing" ANAL. CHEM., no. 69, 1997, pages 4649-4652, XP000727176
- PEARSON J E ET AL : "Surface plasmon resonance: a study of the effect of biotinylation on the selection of antibodies for use in immunoassays" JOURNAL OF IMMUNOLOGICAL METHODS, vol. 221, no. 1-2, December 1998 (1998-12), pages 87-94, XP004153562
- SASAKI S ET AL: "Novel surface plasmon resonance sensor chip functionalized with organic silica compounds for antibody attachment" ANALYTICA CHIMICA ACTA, no. 368, 17 July 1998 (1998-07-17), pages 71-76, XP001037383
- H. MORGAN & D. M. TAYLOR: 'A Surface plasmon resonance immnosensor based on the streptavidin-biotin complex.' BIOSENSORS & BIOELECTRONICS vol. 7, 1992, pages 405 - 410, XP002933655
- Bun Takeyama et. al., "Kakunai juyoutai to koakuchibeetaa no sougo sayou o shihyou to shita hormone you kassei sokuteihou", Dai 24 kai Kankyou Tokisokorojii symposium, Dai 2 kai Eisei Yakugaku Forum Goudou Taikai Kouen Youshishuu (05.98.12) p81, XP002933656

## Description

This invention relates to a method for detecting an antigen, and a biosensor, and more particularly, to a method for detecting an antigen, and a biosensor, the method and the biosensor utilizing surface plasmon resonance.

### RELATED ART

Endocrine disruptors are chemicals which adversely affect the endocrine functions of organisms. When this chemical liberated into the environment enters the body of an organism, it works like a hormone, disrupting the actions of intrinsic hormones (e.g., thyroid hormones and estrogens), and exerting adverse influences on reproductive functions and immune functions of normal organisms and their descendants. "Endocrine disruptors" may be called endocrine-disrupting chemicals or hormonal chemicals.

According to "An Interim Report from the Research Group on the Problem of Exogenous Endocrine-Disrupting Chemicals" (1997) by the Environmental Agency in Japan, endocrine disruptors can be classified, for example, into (a) industrial chemicals (synthetic detergents, paints, cosmetics, plastic plasticizers), (b) dioxins, (c) agrochemicals (herbicides, antifungal agents, insecticides), (d) drugs (synthetic hormones), and (e) naturally occurring substances.

Examples of the industrial chemicals are alkylphenols such as nonylphenol and octylphenol, some bisphenols such as bisphenol A, and phthalic acid derivatives such as butyl benzyl phthalate, and dibutyl phthalate. Examples of the agrochemicals are DDT (DDD, DDE), endosulfan, methoxychlor, heptachlor, toxaphene, dieldrin, and lindane. Examples of the drugs are diethylstilbestrol (DES), and ethynylestradiol (oral contraceptive). Among the naturally occurring substances are soybean, and genistein which is a plant estrogen contained, particularly, in soybean.

Table 1 exemplifies the classification of endocrine disruptors.

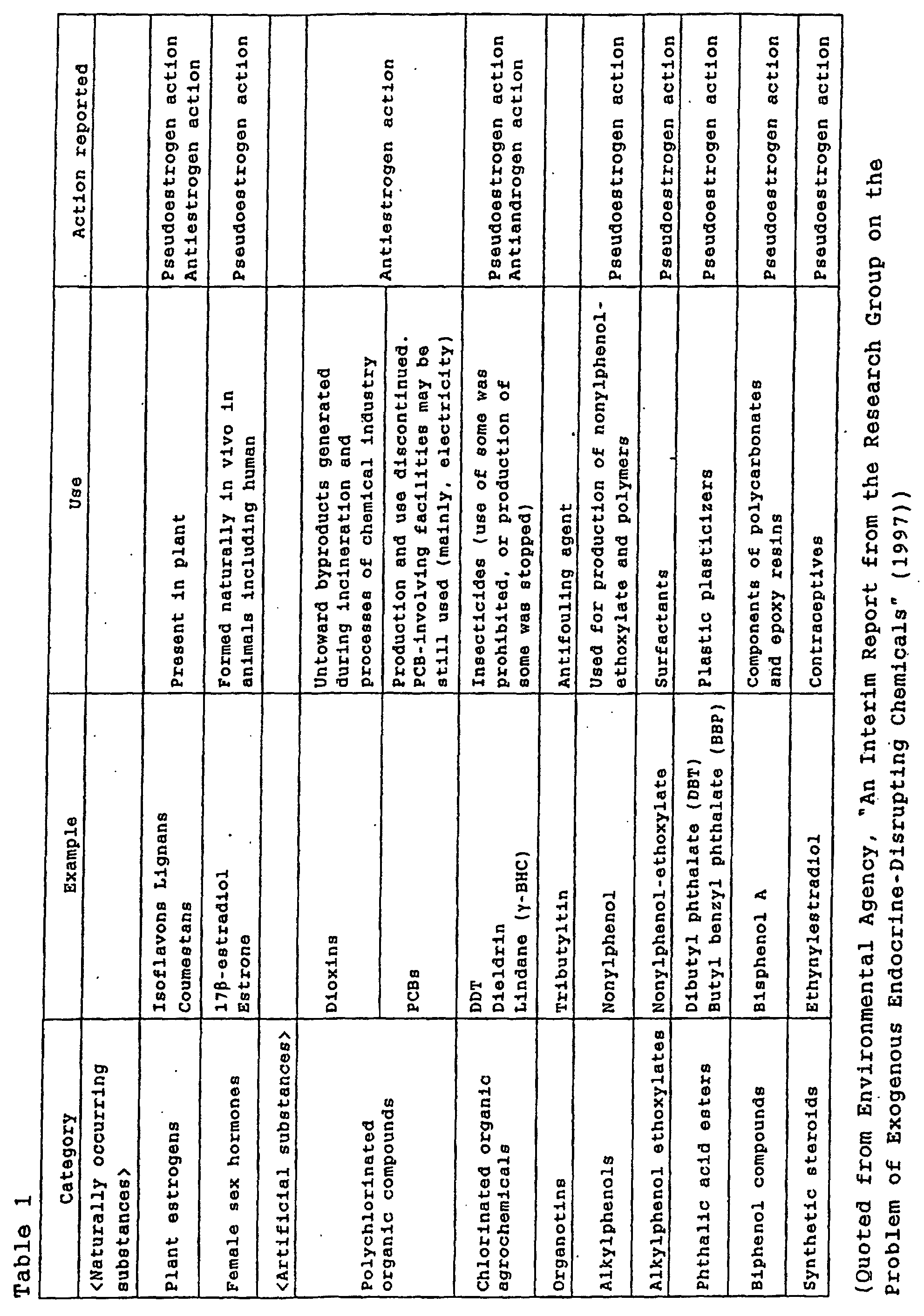

Endocrine disruptors are ingested into the body mainly via foods. Most endocrine disruptors are highly liposoluble, and minimally degradable. Thus, they are apt to be accumulated into the body through the food chain. They are highly accumulated, particularly in birds or marine mammals on an upper level in the food chain. When an endocrine disruptor is accumulated in vivo, it unbalances hormones, causing abnormalities in growth, development, and immune function, or cancer such as breast cancer or testicular cancer, or decreases in spermatozoa. For example, reports have been made of mutation of sea snails by tributyltin, and decreases in the number of alligators in Florida by DDT.

The endocrine disruptors that have attracted the widest attention in recent years are dioxins. Dioxin is a carcinogenic substance with the highest toxicity of all the substances created by human. Dioxin is so toxic that its intake allowance is low.

For example, the Ministry of Health and Welfare in Japan proposed 10 picograms -TEQ kg⁻¹ day⁻¹ as the tolerable daily intake (TDI) of dioxins in June 1996. On the other hand, the Environmental Agency in Japan established 5 picograms -TEQ kg⁻¹ day⁻¹ as the health risk evaluation guideline value in December 1996. Table 2 summarizes the intake allowances of dioxins set by different countries.

**Table 2**

| (As of May 1997) | | |
|---|---|---|
| Country/Organization (time set) | Intake allowance (pg/kg/day) | Remarks |
| Germany (1985) | 1-10 | (prevention level to emergency action level) |
| Sweden (1988) | 0-5 | (0 to 35 per week) |
| Denmark (1988) | 0-5 | (0 to 35 per week) |
| WHO European office (1990) | 10 | |
| Canada (1990) | 10 | |
| Netherlands (1991) | 10 | |
| United Kingdom (1992) | 10 | |
| USA (1994) | 0.01, etc. | (under proposal by EPA, etc.) |
| Netherlands (1996) | 1 | (report from National Health Council) |
| Ministry of Health and Welfare (1996) | 10 | |
| (Quoted from "Risk Evaluation of Dioxin", supervised by Environmental Agency Dioxin Risk Evaluation Study Meeting, Chuo Hoki Publishing, 1997) | | |

Since the intake allowance of dioxins is very low as noted above, it is difficult to measure such trace amounts of dioxins. Also, numerous isomers of dioxins exist, and their analysis is even more difficult. Furthermore, a sample containing dioxins often involves the copresence of dibenzofurans and PCB which are similar in structure to dioxins, and separation from these substances is also demanded.

So far, gas chromatography-mass spectrometry (GC-MS) has been admitted to be the most reliable method for measurement of dioxins. With GC-MS, however, a very complicated pretreatment is required, and the pretreatment takes 2 to 3 weeks, for example.

FIG. 12 shows a flow sheet for pretreatment of a waste water sample. The waste water sample in a suitable amount is filtered through a glass fiber filter paper (pore diameter 1 µm), and thereby divided into filtrate and solids. The filtrate (1 liter) is extracted twice with 100 ml of dichloromethane. The solids are dehydrated with acetone, and then subjected to Soxhlet extraction with toluene for 16 hours or more.

To a mixture of the respective extracts, internal standards 13C-2,3,7,8-TCDD, 13C-2,3,7,8-TCDF, 13C-2,3,7,8-OCDD, and 13C-2,3,7,8-OCDF are added. The mixture is concentrated to about 5 ml by means of a concentrator. As much toluene as possible is distilled off, with a nitrogen gas being blown onto the concentrate, and then the sample is treated with sulfuric acid. The internal standards should desirably be added, where possible, in appropriate ranges of 17 types of isomers, including chlorine-substituted compounds other than the above-mentioned four isomers, and be recovered for correction. After such pretreatment, dioxins are measured with ordinary GC-MS.

As described above, GC-MS has required a very complicated pretreatment, which has been time-consuming. Moreover, a high degree of safety measure has been necessitated to avoid the exposure of an operator to endocrine disruptors in the stage of pretreatment.

Under these circumstances, easy, safe detection of endocrine disruptors such as dioxins is sought for instead of GC-MS.

Publicly known substances may not yet have been established as endocrine disruptors. If a novel substance is synthesized, this novel substance may be an endocrine disruptor.

However, it is not easy to investigate whether or not a certain substance works in vivo in the same or similar manner as or to a hormone to disrupt the endocrine system.

Thus, it is desired to screen easily whether a substance which has not been recognized as an endocrine disruptor is an endocrine disruptor or not. That is, it is desired to conveniently screen a certain substance for a possibility of being an endocrine disruptor, and then to investigate whether a candidate for an endocrine disruptor having passed screening disrupts the endocrine system in vivo or not.

A collection of abstracts of "The Fifth World Congress of Biosensors" held on June 3 to 5, 1998 at InterContinental Hotel in Berlin, Germany describes that atrazine is detected by surface plasmon resonance with the use of a sensor chip having an atrazine antibody fixed thereto. This reference, however, is not silent on antibodies against endocrine disruptors other than atrazine.

Satoshi Sasaki, Ryohei Nagata, Bertold Hock, Isao Karube, Analytica Chimica Acta 368(1998)71-76, dated July 17, 1998, describes that atrazine is detected by surface plasmon resonance with the use of a sensor chip having an atrazine antibody fixed thereto. This reference, however, does not describe antibodies against endocrine disruptors other than atrazine.

### SUMMARY OF THE INVENTION

The present invention can detect an antigen with high sensitivity by use of surface plasmon resonance. Its sensitivity may reach, say, about 0.1 ppb. The invention can also detect the antigen qualitatively and quantitatively. Furthermore, the invention is simple in procedure in comparison with the conventional GC-MS method, or methods using enzyme reactions. Besides, the invention uses an antigen-antibody reaction, and thus is excellent in selectivity.

If the antigen is an endocrine disruptor, the invention can detect the endocrine disruptor. If the antigen is a substance having a steroid skeleton, a substance for maintaining, promoting or inhibiting the physiological action of a steroid hormone, or a substance for maintaining, promoting or inhibiting the physiological action of a sex hormone, the invention is useful for screening of the endocrine disruptor.

If the antigen is a steroid hormone or a sex hormone, the invention is also useful as a medical sensor. For example, the invention can be applied to tests for abnormal secretion of steroid hormone and sex hormone (e.g., test for sterility), test for pregnancy, test for menopausal syndrome, and doping test in athletes.

According to an aspect of the invention, there is provided a method for the use of a sensor chip for surface plasmon resonance, having a metallic thin film; one or more antibodies against one or more antigens selected from a substance having a steroid skeleton, a substance for maintaining, promoting or inhibiting the physiological action of a steroid hormone, a substance for maintaining, promoting or inhibiting the physiological action of a sex hormone, and an endocrine disruptor (excluding a triazine compound); and a fixing layer for fixing the antibody to the metallic thin film.

In the invention, the antibody is preferably an antibody against a steroid hormone, or against a substance showing the same physiological action as that of a steroid hormone. Alternatively, the antibody is preferably an antibody against a sex hormone, or against a substance showing the same physiological action as that of a sex hormone.

More preferably, the antibody is an antibody against an estrogen. Alternatively, the antibody is preferably an antibody against an endocrine disruptor having a cyclic hydrocarbon portion, and more preferably an antibody against dioxins.

Preferably, the antibody is fixed to the fixing layer by a covalent bond. The fixing layer preferably includes one or more organic thin film layers.

The organic thin film layer preferably has a linker layer coating the metallic thin film. Alternatively, the organic thin film layer preferably has a hydrogel layer. Alternatively, the organic thin film layer preferably has a streptavidin layer. Alternatively, the organic thin film layer preferably has a hydrophobic layer. The hydrophobic layer preferably contains a silicon atom which binds to the metallic thin film.

The metallic thin film preferably has an irradiation surface to be irradiated with incident light, and a detection surface on a reverse side to the irradiation surface. The fixing layer preferably coats the detection surface of the metallic thin film. The irradiation surface of the metallic thin film preferably coats a substrate pervious to irradiated light. The sensor chip preferably has two or more of the antibodies against two or more of the antigens.

According to another aspect of the invention, there is provided a method for detecting an antigen, comprising the steps of:
exposing an antibody of a sensor chip for surface plasmon resonance to a sample, the sensor chip having a metallic thin film; one or more of the antibodies against one or more antigens selected from a substance having a steroid skeleton, a substance for maintaining, promoting or inhibiting the physiological action of a steroid hormone, a substance for maintaining, promoting or inhibiting the physiological action of a sex hormone, and an endocrine disruptor (excluding a triazine compound); and a fixing layer for fixing the antibody to the metallic thin film;
irradiating the metallic thin film with light; and
detecting intensity of reflected light from the metallic thin film.

In the invention, the method preferably further includes the step of finding a shift in a disappearance angle of the reflected light. Also, the method preferably further includes the step of binding a labeled antigen, which is the above antigen bound to a labeling material, to the antibody before the exposing step.

The antibody is preferably an antibody against a steroid hormone, or against a substance showing the same physiological action as that of a steroid hormone. Alternatively, the antibody is preferably an antibody against a sex hormone, or against a substance showing the same physiological action as that of a sex hormone. Preferably, the antibody is an antibody against an estrogen.

Alternatively, the antibody is preferably an antibody against an endocrine disruptor having a cyclic hydrocarbon portion. Preferably, the antibody is an antibody against dioxins.

The sensor chip for surface plasmon resonance preferably has two or more of the antibodies against two or more of the antigens, and can detect the two or more antigens.

According to still another aspect of the invention, there is provided a surface plasmon resonance apparatus comprising:
a sensor chip for surface plasmon resonance, having a metallic thin film; one or more antibodies against one or more antigens selected from a substance having a steroid skeleton, a substance for maintaining, promoting or inhibiting the physiological action of a steroid hormone, a substance for maintaining, promoting or inhibiting the physiological action of a sex hormone, and an endocrine disruptor (excluding a triazine compound); and a fixing layer for fixing the antibody to the metallic thin film;
an optical system having a light source for irradiating the sensor chip for surface plasmon resonance with irradiation light; a detector for detecting reflected light from the sensor chip for surface plasmon resonance; and a prism pervious to at least the irradiation light; and
a flow passage system for bringing a sample into contact with the antibody of the sensor chip for surface plasmon resonance.

In the invention, the flow passage system preferably has a flow cell, and the sensor chip for surface plasmon resonance is located at a position corresponding to the flow cell.

### BRIEF DESCRIPTION OF THE DRAWINGS

FIG. 1A is a schematic explanation drawing of a method for measuring an endocrine disruptor according to the present invention;
FIG. 1B is a schematic explanation drawing of the method for measuring an endocrine disruptor according to the invention;
FIG. 2A is a graph showing the correlation between the angle of incidence of irradiated light, i.e., the angle of reflection of reflected light, and the intensity of reflected light;
FIG. 2B is a graph showing the correlation between an elapsed time and a resonance signal;
FIG. 3 is an explanatory sectional view of an embodiment of a sensor chip for surface plasmon resonance according to the invention;
FIG. 4 is an explanatory sectional view of another embodiment of the sensor chip for surface plasmon resonance according to the invention;
FIG. 5A is an exploded sectional view of the sensor chip for surface plasmon resonance of the invention, and an SPR apparatus;
FIG. 5B is a sectional explanation drawing of the sensor chip for surface plasmon resonance of the invention, and the SPR apparatus;
FIG. 6A is an explanation drawing of a flow passage system of the SPR apparatus;
FIG. 6B is an enlarged sectional view of the flow passage system of the SPR apparatus;
FIG. 7 is an enlarged explanation drawing of the flow passage system of the SPR apparatus;
FIG. 8A, FIG. 8B and FIG. 8C are explanation drawings of the competitive method;
FIG. 9 shows the correlation between the dioxin concentration and the response value in the competitive method;
FIG. 10 shows the correlation between the elapsed time after fixing of an anti-dioxin monoclonal antibody and the response value;
FIG. 11 shows the correlation between the elapsed time after fixing of an anti-estradiol monoclonal antibody and the response value;
FIG. 12 is a flow sheet for conventional pretreatment of a waste water sample;
FIG. 13 shows coupling methods; and
FIG. 14 shows·coupling methods.

### PREFERRED EMBODIMENTS OF THE INVENTION

The invention relates to a biosensor, and a method for detecting an antigen, the biosensor and the method utilizing surface plasmon resonance (SPR). Surface plasmon resonance refers to a phenomenon in which when a metallic thin film is irradiated with light, light is transmitted to a surface on a reverse side to the irradiated surface. Conditions under which surface plasmon resonance takes place are determined to be unique.

In the invention, an SPR apparatus, called BIACORE (registered trademark), which is produced by Pharmacia Biosensor (now, Biacore), Sweden, can be used preferably. In Japan, this apparatus can be obtained from Biacore Kabushiki Kaisha (3-25-1, Hyakunin-cho, Shinjuku-ku, Tokyo 169-0073, Japan).

The principle of measurement, and the method for measurement, by a biosensor which utilizes surface plasmon resonance, and BIACORE are described in Hashimoto, S., "Analysis of biomolecule interaction utilizing surface plasmon resonance phenomenon", Bunseki 1997(5)362-368; Edited by Nagata, K. and Handa, H., "Methods for real time analysis experiments on biomaterial interaction - Focused on BIACORE", Schpringer Fairlark Tokyo, 1998; and Kawada, S. (1989) "Surface plasmon sensor" 0 plus E, 112, 133-139. These descriptions are cited in the specification of the present application.

First of all, the principle of surface plasmon resonance is explained with reference to FIG. 1A. In FIG. 1A, a sensor chip 10 includes a metallic thin film, and an antibody 19 against a predetermined antigen is fixed to one surface of the sensor chip 10. The antibody 19 is exposed to a flow passage 22 through which a sample optionally containing a predetermined antigen 29 flows. The sample is a fluid, and typically a liquid.

FIG. 3 is an explanatory sectional view of the sensor chip 10. In FIG. 3, the sensor chip 10 includes a metallic thin film 14, and the metallic thin film 14 has an irradiation surface 14s to be irradiated with incident light, and a detection surface 14t on the reverse side to the irradiation surface 14s. In other words, the metallic thin film 14 has the detection surface 14t on the side where the antibody 19 is fixed, and the irradiation surface 14s on the opposite side.

In FIG. 1A, a prism 34 is disposed on a surface 10s of the sensor chip 10. A light source 32 projects light which passes through the prism 34 to irradiate the irradiation surface 14s of the metallic thin film 14 of the sensor chip 10. This light is preferably polarized light, and its wavelength is preferably 300 to 2,000 nm. A detector 36 monitors the intensity of reflected light which has been reflected from the irradiation surface 14s of the metallic thin film 14 of the sensor chip 10 and passed through the prism 34. The angle of incidence of the irradiated light and the angle of reflection of the reflected light equal without doubt. At the metallic thin film, usually 90% or more of the incident light is reflected, and the remainder penetrates through the metallic thin film.

FIG. 2A shows the correlation between the angle of incidence of irradiated light, i.e., the angle of reflection of reflected light, and the intensity of reflected light. When light is projected with its angle of incidence being varied, there is an angle at which the intensity of reflected light attenuates, as shown by a "valley of light" 42 on a curve 40. This angle is called the disappearance angle or SPR angle of reflected light. At this disappearance angle, a quantum mechanical phenomenon called surface plasmon resonance occurs on the detection surface 14t of the metallic thin film 14. More correctly, a wave propagating only through the detection surface 14t of the metallic thin film 14 (this wave is called an evanescent wave) is generated to induce a surface plasmon of free electrons of the metallic thin film. Part of the energy of the irradiated light is converted to the energy of surface plasmon resonance, and in accordance with this phenomenon, the intensity of reflected light decays.

This disappearance angle varies depending on the state of the detection surface 14t of the metallic thin film 14, i.e., the surface 14t of the metallic thin film 14 on the side where the antibody 19 is fixed, e.g., depending on whether the predetermined antigen 29 has been found to the antibody 19. According to an embodiment of the method of the invention, therefore, a shift in the disappearance angle is measured.

When the sample containing the predetermined antigen 29 is flowed through the flow passage 22 as shown in FIG. 1A, the predetermined antigen 29 binds to the antibody 19 on the surface of the sensor chip 10 as indicated in FIG. 1B.

According to an embodiment of the method of the invention, a change in the disappearance angle is measured as shown in FIG. 2A. According to another embodiment of the method of the invention, the time course of a resonance signal at a certain angle, typically disappearance angle, is measured as shown in FIG. 2B.

In FIG. 2A, the curve 40 shows the correlation between the angle of incidence of incident light and the intensity of reflected light in a state in which the predetermined antigen 29 has not bound to the antibody 19. A curve 44, on the other hand, shows the correlation between the angle of incidence of incident light and the intensity of reflected light in a state in which the predetermined antigen 29 has bound to the antibody 19. One will see that as a result of binding of the predetermined antigen 29 to the antibody 19, "the valley of light" 42 moves to "a valley of light" 46. That is, the binding of the predetermined antigen 29 to the antibody 19 results in a shift in the disappearance angle. The degree of the shift in the disappearance angle is correlated with the amount of the predetermined antigen bound to the antibody 19, i.e., the amount of the predetermined antigen in the sample.

As a unit representing the degree of movement of "the valley of light", a change of 0.1 degree in the SPR angle is defined as 1,000 resonance units (RU). Experiments were conducted by use of a radioisotope-labeled protein to observe the correlation between an SPR signal and the protein concentration on the sensor chip surface. These experiments have confirmed that 1,000 RU corresponds to a mass change of about 1 ng/mm² in the protein on the sensor chip surface. This value is almost constant regardless of the type of protein. In actual measurements, a change of about 10 RU (about 10 pg/mm²) can be observed.

FIG. 2B shows the correlation between time and the intensity of reflected light at "the valley of light" 42, i.e., the disappearance angle. In other words, FIG. 2B shows the time course of the intensity of reflected light at the disappearance angle 42. It is seen that as the predetermined antigen 29 binds to the antibody 19, the disappearance angle varies, and surface plasmon resonance occurs no more. The interaction between the antibody 19 and the predetermined antigen 29 on the surface of the sensor chip 10 can be monitored in real time.

FIG. 3 shows an embodiment of a sensor chip for surface plasmon resonance according to the invention. U.S. Patent 5,242,828 describes a detection surface for a biosensor. This detection surface for a biosensor can be used preferably for the sensor chip for surface plasmon resonance of the invention.

The sensor chip 10 preferably has a substrate 12 pervious to light. As the substrate 12, glass is typically used. As the substrate, a material of an optical fiber may be used. For example, a glass slide 0.2 to 0.8 mm thick can be used.

The sensor chip 10 has the metallic thin film 14, because surface plasmon resonance occurs in the detection surface 14t of the metallic thin film 14 when light is projected onto the irradiation surface 14s of the metallic thin film 14 under predetermined conditions. When the sensor chip 10 is to be produced, the metallic thin film 14 is formed, for example, on the glass substrate 12 by vapor deposition, sputtering, or electroless plating. Vacuum deposition may be physical vacuum deposition or chemical vacuum deposition.

The metallic thin film 14 may have a single-layer structure, or a multilayer structure such as a two-layer structure. In FIG. 3, the metallic thin film 14 has a single-layer structure.

The thickness of the metallic thin film 14 is preferably 10 to 100 nm, more preferably 20 to 80 nm, and even more preferably 20 to 60 nm. If the thickness is larger than 100 nm, surface plasmon resonance minimally occurs. If the thickness is less than 10 nm, it becomes difficult to form a uniform thickness. To improve the reproducibility of SPR signal, the thickness of the metallic thin film 14 is preferably uniform.

The metallic thin film 14 preferably comprises gold, silver, copper, aluminum, platinum or iridium, or preferably comprises a noble metal such as gold, silver or platinum. The irradiation surface 14s, in particular, preferably comprises a noble metal. This is because a noble metal is chemically inert and has a high efficiency of generating an SPR signal. Of noble metals, silver is preferred from aspects, such as chemical stability and high SPR signal generating efficiency.

The metallic thin film 14 may be composed of not less than 80% by weight, preferably not less than 90% by weight, of a noble metal, and not more than 20% by weight, preferably not more than 10% by weight, of other metal. Examples of the other metal are typical metals such as aluminum, and transition metals such as chromium.

The sensor chip 10 has a fixing layer 16 for fixing the antibody 19 to the metallic thin film 14. The fixing layer 16 is formed on the detection surface 14t of the metallic thin film 14, because it is difficult to fix the antibody 19 directly to the metallic thin film 14.

The fixing layer 16 may have a multilayer structure such as a two-layer structure, or may have a single-layer structure. In FIG. 3, the fixing layer 16 has a two-layer structure composed of a linker layer 17 and a fixing body 18. The linker layer 17 preferably coats the detection surface 14t of the metallic thin film 14, and as described in the aforementioned U.S.P. 5,242,828, preferably can protect the detection surface 14s of the metallic thin film 14 from corrosion, etc.

The fixing body 18, on the other hand, serves to fix the antibody 19 stably. The antibody 19 is preferably fixed to the fixing body 18 by a covalent bond. Also, the fixing body 18 is preferably bound to the linker layer 17 by a covalent bond.

An example of the linker layer 17 is, as described in USP 5,242,828, a single layer of an organic molecule expressed by the formula X-R-Y where X denotes disulfide, sulfide, selenide, thiol, nitrile, or isonitrile.

R denotes a hydrocarbon group with 12 to 30 carbon atoms which may be interrupted by a hetero-atom such as an oxygen atom. R is, for example, an alkylene group.

Y is an active group for binding to the antibody 19, such as a hydroxyl group, carboxyl group, amino group, aldehyde group, hydrazide group, carbonyl group, epoxy group, or vinyl group.

As the X-R-Y, 16-mercaptohexadecanol, for example, is used.

As the fixing body 18, hydrogel may be used as described in USP 5,436,161. As hydrogen, polysaccharides and organic polymers can be used preferably. The polysaccharides include agarose, dextran, or their carboxymethylated derivatives. Examples of the organic polymers are polyvinyl alcohol, polyacrylic acid, polyacrylamide, and polyethylene glycol.

As the hydrogel, dextran or its derivative such as carboxymethyldextran is preferred. Particularly, single-chain dextran having no crosslinked structure, or its derivative such as carboxymethyldextran is preferred. The use of the dextran or its derivative brings the following advantages:
① By using a chemical reaction in wide use, the antibody 19 can be fixed by a covalent bond.
② The volume of the antibody 19 bound can be increased.
③ A flexible, hydrophilic matrix environment suitable for the interaction between the antibody 19 and the predetermined antigen 29 can be provided.
④ Nonspecific binding to the sensor chip surface can be kept low.

To facilitate the observation of SPR signal, the thickness of the fixing body 18 is preferably 1 to 3 times the thickness of the metallic thin film 15. The thickness of the fixing body 18 is preferably 50 to 200 nm, and more preferably 60 to 150 nm. For the reproducibility of SPR signal, the thickness of the fixing body is preferably uniform.

FIG. 4 shows another embodiment of the sensor chip for surface plasmon resonance according to the invention. The same constituent elements are assigned the same reference numerals, and their explanations are omitted. With the sensor chip of FIG. 4, a metallic thin film 14 has a two-layer structure. A transition metal thin film 15a of chromium or the like is formed on a substrate 12. On the transition metal thin film 15a, a noble metal thin film 15b is formed. The transition metal thin film 15a is preferably thinner than the noble metal thin film 15b. The thickness of the transition metal thin film 15a is preferably not more than 30%, more preferably not more than 20%, of the thickness of the metallic thin film 14.

In FIG. 4, a fixing layer 16 does not have a two-layer structure, but has a single-layer structure. In an embodiment, an organosilicon compound having a functional group, such as γ-aminopropylethoxysilane, or (NH₂CH₂CH₂CH₂)(CH₃CH₂O)SiH₂, is used, whereby the fixing layer 16 can be stably bound to the metallic thin film 14 via the silicon atom. Also, the amino group of the organosilicon compound is reacted with aldehyde, and then the antibody 19 is introduced, whereby a covalent bond can be formed between the fixing layer 16 and the antibody 19.

The fixing layer and the antibody preferably have a portion represented by the formula Si-L-Z-A
where L is a hydrocarbon group with 1 to 18 carbon atoms which may be interrupted by a hetero-atom such as an oxygen atom,
A is the antibody, and
Z is a bond formed by the coupling of the active group of the fixing layer to the antibody.

L is preferably a group of the formula -(CH₂)ₙ- where n is an integer of 1 to 18, preferably 2 to 10.

Examples of the Z are a thiol bond (-SS-), an acid amide bond (-C(=O)NH-), a streptavidin-biotin bond, and a bond of the formula -C(=O)-N(H)N=C-.

In another embodiment of FIG. 4, the fixing layer 16 may be a thiolalkane group, which makes it possible to form a very hydrophobic surface and reproduce a state similar to a biomembrane on the sensor surface. For example, the fixing layer 16 having a single-layer structure can be formed by forming the aforementioned linker layer with a large thickness.

The fixing layer may have a three-layer structure. For example, streptavidin may be bound, by amine coupling, onto the fixing body 18 of the sensor chip for surface plasmon resonance described in FIG. 3 to form a streptavidin layer. For example, the fixing layer may be formed by laminating the linker layer 17, hydrogel layer, and streptavidin layer in this order.

Streptavidin is a protein having a tetrameric structure with a molecular weight of 60,000. Since the dissociation constant for the binding of streptavidin-biotin is 10¹⁵M, the streptavidin layer can fix the biotinylated antibody very stably.

Furthermore, various layers can be formed in the fixing layer by publicly known coupling methods, as shown in FIGS. 13 and 14, to design a multilayer structure.

In the sensor chip of the invention, a predetermined antibody 19 is fixed to the fixing layer 16. First, the predetermined antigen will be described, and then the antibody 19 against it will be explained.

According to the invention, a substance having a steroid skeleton, a substance for maintaining, promoting or inhibiting the physiological action of a steroid hormone, a substance for maintaining, promoting or inhibiting the physiological action of a sex hormone, or an endocrine disruptor (excluding a triazine compound) serves as an antigen. A typical example of the substance for maintaining, promoting or inhibiting the physiological action of a steroid hormone, especially the substance for maintaining the physiological action of a steroid hormone, is a steroid hormone. Similarly, a typical example of the substance for maintaining, promoting or inhibiting the physiological action of a sex hormone, especially the substance for maintaining the physiological action of a sex hormone, is a sex hormone.

A certain substance may be a substance having a steroid skeleton, a steroid hormone, and at the same time, a sex hormone. For example, estradiol-17β is a substance having a steroid skeleton, a steroid hormone, and at the same time, a sex hormone. Moreover, a certain substance may be a substance for maintaining, promoting or inhibiting the physiological action of a steroid hormone, a substance for maintaining, promoting or inhibiting the physiological action of a sex hormone, and at the same time an endocrine disruptor (excluding a triazine compound). In the invention, it is an object to detect a substance which falls under any of the above categories.

For example, when an antibody against estradiol-17β is fixed to the sensor chip, estradiol-17β in a sample can be detected by surface plasmon resonance. A substance other than estradiol-17β may be bound to the antibody against estradiol-17β. By use of surface plasmon resonance, it can be detected whether a certain substance has bound to the antibody against estradiol-17β.

A substance binding to the antibody against estradiol-17β on the sensor chip is likely to bind to a receptor of estradiol-17β in vivo, disrupting the endocrine system. That is, such a substance may show a physiological action similar to that of estradiol-17β, and may fit into the category of endocrine disruptors. Thus, detection of whether or not a certain substance binds to the antibody against estradiol-17β serves as screening of whether this substance is an endocrine disruptor or not.

The principle of screening for an endocrine disruptor is applicable not only to substances having a physiological action similar to that of estradiol-17β, but also to other substances showing a physiological action similar to that of other steroid hormones and sex hormones.

In addition, the fact that a certain substance binds to an antibody against dioxins suggests that this substance may show a physiological action similar to that of dioxins in vivo.

In consideration of these facts, the principle of screening can be applied equally to a substance having a steroid skeleton, a substance for maintaining, promoting or inhibiting the physiological action of a steroid hormone, and a substance for maintaining, promoting or inhibiting the physiological action of a sex hormone. A further detailed description of the antigen will be offered below.

Steroid is a generic name for a group of compounds having a steroid skeleton, i.e., a cyclopentanoperhydrophenanthrene carbon skeleton. The structure of the steroid skeleton is shown below.

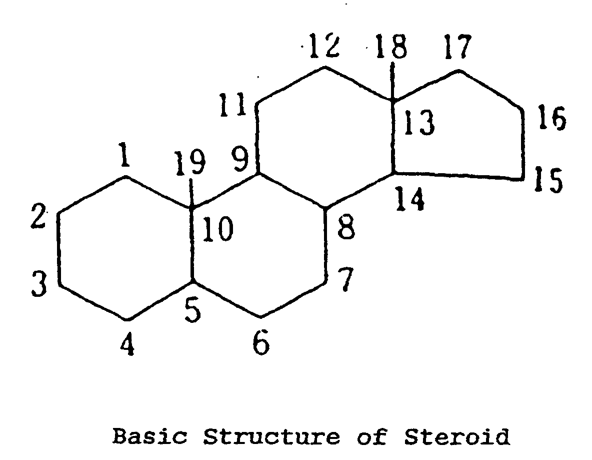

Among steroids, sterols, bile acids, sex hormones, and adrenocortical hormones are included. Sex hormones are steroids in many cases, but may include non-steroidal hormones like estrogenic hormones.

Among C₁₈ steroids, estrogens are named. Examples of C₁₉ steroids are androgens. Examples of C₂₁ steroids are gestagens and adrenal corticoids. Examples of C₂₄ steroids are bile acids.

Adrenal corticoids are roughly classified into glucocorticoids and mineralocorticoids. Among glucocorticoids are cortisol, cortisone, and corticosterone. Among mineralocorticoids, aldosterone, 11-deoxycorticosterone, and 11-deoxycortisol are included.

Many bile acids in higher vertebrates such as mammals are hydroxy derivatives of cholanic acid. Human bile contains cholic acid, deoxycholic acid, chenodeoxycholic acid, and lithocholic acid. Depending on the species of animals, the types and composition of bile acid slightly differ.

Among substances having a steroid skeleton, tetrodotoxin is included. Since tetrodotoxin is a deadly poison, simple detection of a trace amount of tetrodotoxin is meaningful.

Steroid hormones refer to hormones which are steroids, and their examples are estrogens, androgens, gestagens, and adrenal corticoids.

Sex hormones generically refer to hormones which are secreted mainly from gonads to induce and promote the growth and development of the reproductive system, and also arouse secondary sexual characters and reproductive behaviors. Among sex hormones, androgens, estrogens and gestagens are included.

Androgens generally refer to steroid hormones having androgenic hormone activity. In the present specification, however, androgens refer to steroids having an androstane skeleton with 19 carbon atoms. The androstane skeleton is shown below.

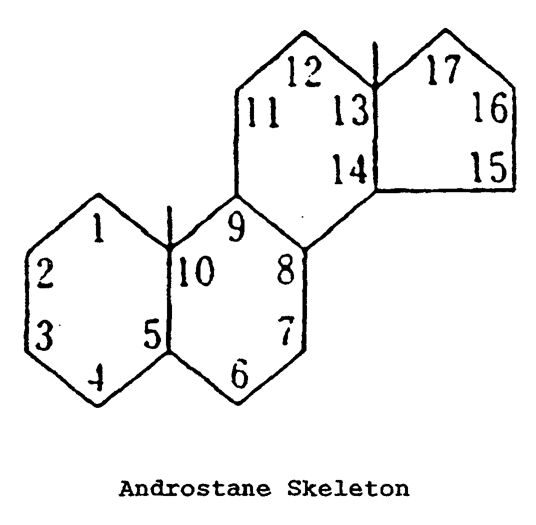

Among androgens, testosterone, androstenedione, and dehydroepiandrosterone are included.

The physiological actions of androgen include, for example, sexual differentiation in the embryonic stage, maintenance of function of male genital organs (ductus deferens, prostate, seminal vesicles, epididymis, external genitalia), development of secondary sexual characters, promotion of spermatogenesis, and promotion of protein anabolic action in skeletal muscles. Another example is to act on the hypothalamus and hypophysis to suppress luteinizing hormone secretion by the negative feedback mechanism.

For example, testosterone shows the following physiological actions: Testosterone has the functions of developing secondary sexual characters, showing protein anabolic action, and promoting growth of muscles. It also acts on the hypophysis to suppress secretion of gonadotropic hormones, and also shows the physiological action of keeping the testosterone concentration in the blood constant by the feedback mechanism.

Estrogen is a type of sex hormone which is secreted mainly from the ovary in vertebrates to grow female gonadal appendages for their full functions. In mammals, estrogen induces an estrous state. Estrogen is secreted from the placenta as well, and is also secreted in small amounts from the adrenal cortex and testis. Estrogen includes estrogenic hormones and corpus luteum hormones. Main estrogens are estradiol-17β, estrone, and estriol.

Estrogens are roughly classified into 1) steroid hormones and their metabolites, such as estradiol synthesized in and secreted from a mature ovarian follicle of the ovary and the placenta in pregnancy, and estrone, estriol, equilin and equilenin found in large amounts in urine, etc., 2) chemical derivatives of these hormones having estrogenic action (e.g., homoestrone, ethynylestradiol, doisynolic acid), and 3) synthetic substances having no steroid structure, but showing estrogenic action, i.e., synthetic estrogens (e.g., diethylstilbestrol, hexestrol).

For examples, estradiol-17β shows the following physiological actions: It promotes the growth of an ovarian follicle and the mammary gland, and acts on adnexal gonads, accelerating secondary sexual characteristics. It also acts on the arcuate nuclei to induce a negative feedback to luteinizing hormone (LH). However, it works positively in the front area of the fasciculus opticus to secrete gonadotropin-releasing hormone (GnRH), and also increases the sensitivity of the hypophysis to GnRH, causing LH surge before ovulation. In birds, estradiol-17β promotes the synthesis and secretion of egg white protein in the oviduct. In oviparous vertebrates, estradiol-17β acts on the liver to cause transport of vitellogenin, a yolk protein precursor, to the ovary by bloodstream so that vitellogenin is taken into the egg to become yolk protein.

Gestagen is a generic term for compounds having progesterone-like actions, such as implantation of a fertilized egg and maintenance of pregnancy in mammals. Gestagen is also called progestin or progestogen. A typical example of gestagen is progesterone, but steroids resembling progesterone, which do not have the above activities, such as 20β-dihydroxyprogesterone and pregnanediol, are also included in gestagens. Gestagen is secreted from the corpus luteum that has been subjected to the action of corpus luteum hormone.

The physiological action of gestagen is to work in cooperation with estrogen to cause endometrial thickening and uterine gland branching, thereby stimulating the implantation of the fertilized egg. Another action is to act in antagonism to estrogen to suppress estrus, permitting continuation of pregnancy.

Gestagen is secreted from the corpus luteum of lactation as well, and depending on species, it is secreted in large amounts during pregnancy. Gestagen acts on the anterior lobe of the hypophysis in cooperation with estrogen to suppress the secretion of luteinizing hormone. Thus, no ovulation occurs while the corpus luteum is working.

Endocrine disruptors are chemical substances which adversely affect the endocrine function of a living being, as stated earlier. When a chemical substance released into the environment enters the body of an organism, it performs a similar function to that of a hormone to disrupt the actions of intrinsic hormones (e.g., thyroid hormones and estrogens), and exert adverse influences on the reproductive function, immune function, etc. of sound organisms and their descendants.

Examples of endocrine disruptors are dioxins, polychlorinated biphenyls (PCB), polybrominated biphenyls (PCB), hexachlorobenzene (HCB), pentachlorophenol (PCP), 2,4,5-trichlorophenoxyacetic acid, 2,4-dichlorophenoxyacetic acid, amitrole, atrazine, alachlor, simazine, hexachlorocyclohexane, ethylparathion, carbaryl, chlordane, oxychlordane, trans-nonachlor, 1,2-dibromo-3-chloropropane, DDT, DDE, DDD, kelthane, aldrin, endrin, dieldrin, endosulfan (benzoepin), heptachlor, heptachlor epoxide, malathion, methomyl, methoxychlor, mirex, nitrophen, toxaphene, tributyltin, triphenyltin, trifluralin, alkylphenol (C4 to C9), bisphenol A, di-2-ethylhexyl phthalate, butyl benzyl phthalate, di-n-butyl phthalate, dicyclohexyl phthalate, diethyl phthalate, benzo(a)pyrene, 2,4-dichlorophenol, di-2-ethylhexyl adipate, benzophenone, 4-nitrotoluene, octachlorostyrene, aldicarb, benomyl, kepone (chlordecone), manzeb (mancozeb), maneb, methyram, metribuzin, cypermethrin, esfenvalerate, fenvalerate, permethrin, vinclozolin, zineb, ziram, dipentyl phthlate, dihexyl phthlate, dipropyl phthlate, dimer and trimer of styrene, styrene, n-butylbenzene, and estradiol. Of these endocrine disruptors, atrazine and simazine are named as triazine compounds.

Of the endocrine disruptors having a cyclic hydrocarbon portion, some are highly toxic. The cyclic hydrocarbon portion may be aromatic or aliphatic. Examples of the cyclic aromatic hydrocarbon portion are benzene ring, naphthalene ring, phenanthrene ring, anthracene ring, and pyrene ring. For example, indene, phenol, and styrene have an aromatic cyclic hydrocarbon portion, a benzene ring. The cyclic hydrocarbon portion may be a fundamental component of a condensed ring.

Examples of the endocrine disruptors having a cyclic hydrocarbon portion are dioxins, polychlorinated biphenyls (PCB), polybrominated biphenyls (PCB), hexachlorobenzene (HCB), pentachlorophenol (PCP), 2,4,5-trichlorophenoxyacetic acid, 2,4-dichlorophenoxyacetic acid, triphenyltin, alkylphenol (C4 to C9), bisphenol A, phthalic acid alkyl esters, benzo(a)pyrene, 2,4-dichlorophenol, benzophenone, nitrotoluene, octachlorostyrene, dimer and trimer of styrene, styrene, n-butylbenzene, and estradiol.

Among the endocrine disruptors having a cyclic hydrocarbon portion substituted by a halogen atom, especially those having a cyclic aromatic hydrocarbon portion substituted by a halogen atom, the ones with particularly high toxicity are included. The halogen atom refers to a fluorine atom, a chlorine atom, a bromine atom or an iodine atom, or an arbitrary combination of these. Chlorine and bromine atoms, in particular, pose problems. Examples of the endocrine disruptors having a cyclic hydrocarbon portion substituted by a halogen atom are dioxins, polychlorinated biphenyls (PCB), polybrominated biphenyls (PCB), hexachlorobenzene (HCB), pentachlorophenol (PCP), 2,4,5-trichlorophenoxyacetic acid, 2,4-dichlorophenoxyacetic acid, and 2,4-dichlorophenol.

Dioxins refer generically to polychlorodibenzoparadioxin (hereinafter called PCDD) and polychlorodibenzofuran (hereinafter called PCDF). The structural formulas of these compounds are shown below.

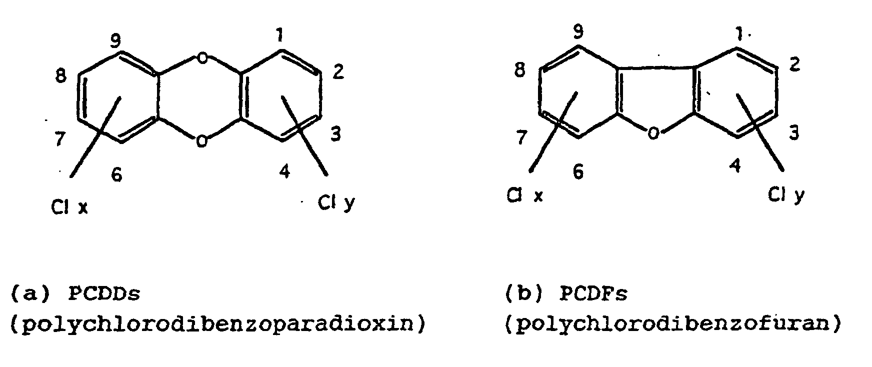

PCDD and PCDF are each a condensed ring including two benzene rings. Since 1 to 8 chlorine atoms are linked to the 1- to 9-carbon positions, 75 isomers exist for PCDD, and 135 isomers for PCDF.

Of these isomers, 2,3,7,8-PCDD has been found to have the highest toxicity. 2,3,7,8-PCDD (molecular weight 321.9) has a melting point of about 300°C. At ordinary temperatures, it is a stable substance, and acid- and alkali-resistant. However, this compound is easily decomposable with ultraviolet radiation, and this nature is utilized in the disposal of PCDD and PCDF.

Dioxins are sparingly soluble in water (2x10⁻⁷ g L⁻¹), but particularly easily soluble in o-dichlorobenzene (1.8 g L⁻¹) with similar polarity. They are soluble in methanol, chloroform, nonane, and toluene. The maximum absorption spectrum is 310 nM when dissolved in chloroform. Such liposolubility of dioxins affects the in vivo accumulation distribution of dioxin incorporated into the body.

The toxicity of the isomers of dioxins is expressed as the titer calculated based on the toxicity of 2,3,7,8-TCDD in view of the binding to the receptor, allylhydrocarbon hydroxylase inducing ability, and toxic effect.

**Table 3**

| No. of chlorine atoms | Name of compound | TEF | Name of compound | TEF |
|---|---|---|---|---|
| 4 | Tetra CDD | | Tetra CDF | |
| | 2,3,7,8- | 1 | 2,3,7,8- | 0.1 |
| | 1,3,6,8- | 0 | 1,3,6,8- | 0 |
| | 1,3,7,9- | 0 | Other | 0 |
| | Other | 0 | | |
| 5 | Penta CDD | | Penta CDF | |
| | 1,2,3,7,8- | 0.5 | 1,2,3,7,8- | 0.05 |
| | Other | 0 | 2,3,4,7,8- | 0.5 |
| | | | Other | 0 |
| 6 | Hexa CDD | | Hexa CDF | |
| | 1,2,3,4,7,8- | 0.1 | 1,2,3,4,7,8- | 0.1 |
| | 1,2,3,6,7,8- | 0.1 | 1,2,3,6,7,8- | 0.1 |
| | 1,2,3,7,8,9- | 0.1 | 1,2,3,7,8,9- | 0.1 |
| | Other | 0 | 2,3,4,6,7,8- | 0.1 |
| | | | Other | 0 |
| 7 | Hepta CDD | | Hepta CDF | |
| | 1,2,3,4,6,7,8- | 0.01 | 1,2,3,4,6,7,8- | 0.1 |
| | Other | 0 | 1,2,3,4,7,8,9- | 0.1 |
| | | | Other | 0 |
| 8 | Octa CDD | 0.001 | Octa CDF | 0.001 |

Next, antibodies against these antigens will be explained. The antibodies are commercially available, or can be prepared experimentally.

As for anti-androgen antibodies, anti-estrogen antibodies, anti-gestagen antibodies, anti-corticosteroid antibodies, and anti-bile acid antibodies, the following antibodies can be obtained from Cosmobio Kabushiki Kaisha (Toyo 2-2-20, Koto-ku, Tokyo 135-0016, Japan):

Examples of the anti-androgen antibodies are anti-testosterone antibody, anti-androstenedione antibody, anti-dehydroepiandrosterone antibody, anti-dihydrotestosterone antibody, anti-19-OH-testosterone antibody, anti-19-OH-androstenedione antibody, anti-11-oxo-testosterone antibody, anti-19-nor-testosterone antibody, anti-19-nor-4-androstenedione antibody, anti-16α-OH-4-androstenedione antibody, anti-16α-OH-dehydroepiandrosterone antibody, anti-16α-OH-dehydroandrosterone antibody, anti-16α-OH-testosterone antibody, anti-5α-androstane-3α,17β-diol antibody, anti-testosterone-glucuronide antibody, anti-androstenedione antibody, anti-11β-OH-androstenedione antibody, anti-5α-androstane-3α,17β-diol-3-glucuronide antibody, and anti-5α-androstane-3α,17β-diol-17-glucuronide antibody.

Examples of the anti-estrogen antibodies are anti-estrone antibody, anti-estradiol antibody, anti-estriol antibody, anti-estrol antibody, anti-16α-OH-estrone antibody, anti-2-OH-estrone antibody, anti-4-OH-estrone antibody, anti-2-methoxyestrone antibody, anti-4-methoxyestrone antibody, anti-ethynylestradiol antibody, anti-estrone-3-glucuronide antibody, anti-estrone-3-sulfate antibody, anti-equilenin antibody, anti-equilin antibody, anti-diethylstilbestrol antibody, and anti-estrone-3 antibody.

Examples of the anti-gestagen antibodies are anti-progesterone antibody, anti-16α-OH-progesterone antibody, anti-17α-OH-progesterone antibody, anti-20α-OH-progesterone antibody, anti-20β-OH-progesterone antibody, anti-pregnenolone antibody, anti-pregnenolone-3 antibody, anti-16α-OH-pregnenolone antibody, anti-17α-OH-pregnenolone antibody, anti-5α-pregnane-3-20-dione antibody, anti-17α-OH-pregnenolone-3-sulfate antibody, anti-17,20α-diOH-progesterone antibody, anti-17α,20β-diOH-progesterone antibody, anti-pregnanediol-3-glucuronide antibody, anti-pregnanediol-3-CMO antibody, anti-pregnanetriol-3-glucuronide antibody, and anti-17α,20β-21-triOH-progesterone antibody.

Examples of the anti-corticosteroid antibodies are anti-cortisol antibody, anti-cortisone antibody, anti-deoxycortisone antibody, anti-corticosterone antibody, anti-deoxycorticosterone antibody, anti-18-OH-corticosterone antibody, anti-18-OH-deoxycorticosterone antibody, anti-aldosterone antibody, anti-11-dehydrocorticosterone antibody, anti-6β-OH-cortisol antibody, anti-THF (tetrahydrocortisol) antibody, anti-THE (tetrahydrocortisone) antibody, anti-THS (tetrahydrodeoxycortisone) antibody, anti-tetrahydro-aldosterone antibody, anti-cortol antibody, anti-cortolone antibody, and anti-11-deoxycortol antibody.

Examples of the anti-bile acid antibodies are anti-cholic acid antibody, anti-glycocholic acid antibody, anti-deoxycholic acid antibody, anti-glycodeoxycholic acid antibody, anti-chenodeoxycholic acid antibody, anti-glycochenodeoxycholic acid antibody, anti-lithocholic acid antibody, anti-glycolithocholic acid antibody, anti-lithocholic acid sulfate antibody, anti-glycolithocholic acid sulfate antibody, anti-chenodeoxycholic acid sulfate antibody, anti-ursodeoxycholic acid antibody, and anti-hyodeoxycholic acid antibody.

An anti-growth hormone polyclonal antibody can be obtained from Cosmobio. Growth hormone is one of steroid hormones.

Commercially available antibodies against endocrine disruptors will be enumerated below. Anti-heptachlor antibody can be obtained from Biostride and Chemicon. Anti-malathion antibody and anti-parathion antibody can be obtained from Chemicon. Anti-PCB antibody can be obtained from Concept.

Methods for preparing anti-dioxin antibodies are described, for example, in L.H. Stanker, B. Walkins, N. Rogers, and M. Vanderlaan, "Anti-dioxin monoclonal antibody: Characterization and assay development of antibody" Toxicology, 45(1987)229-243.

As will be described below, the binding product between an antigen hapten and a polymeric compound can be prepared, and then a polyclonal antibody can be prepared. If desired, a monoclonal antibody can be prepared from the polyclonal antibody.

### Preparation of binding product between antigen hapten and polymeric compound

If the molecular weight of an antigen is small, it is preferred to bind the antigen hapten to a suitable polymeric compound, and then use the binding product as an antigen for immunization.

Preferred examples of the polymeric compound are keyhole-limpet hemocyanin (hereinafter called "KLH"), ovalbumin (hereinafter called "OVA"), bovine serum albumin (hereinafter called "BSA"), and rabbit serum albumin (hereinafter called "RSA").

The binding of the antigen hapten and the polymeric compound can be performed by a publicly known method, such as the activated ester method (A.E. KARU et al.: J. Agric. Food Chem. 42 301-309 (1994)), or the mixed acid anhydride method (B.F. Erlanger et al.: J. Biol. Chem. 234 1090-1094 (1954)).

The activated ester method can be generally carried out in the following manner: First, a hapten compound is dissolved in an organic solvent, and reacted with N-hydroxysuccinimide in the presence of a coupling agent to form an N-hydroxysuccinimide activated ester.

As the coupling agent, an ordinary coupling agent in customary use for a condensation reaction can be used. For example, dicyclohexylcarbodiimide, carbonyldiimidazole, and water-soluble carbodiimide are included. As the organic solvent, dimethyl sulfoxide (hereinafter, "DMSO"), N,N-dimethylformamide (DMF), or dioxane, for example, can be used. The mol ratio between the hapten compound and the N-hydroxysuccinimide used in the reaction is preferably 1:10 to 10:1, more preferably 1:1 to 1:10, and most preferably 1:1. The reaction temperature is 0°C to 100°C, preferably 5°C to 50°C, more preferably 22°C to 27°C. The reaction time is 5 minutes to 24 hours, preferably 30 minutes to 6 hours, more preferably 1 hour to 4 hours. The reaction temperature may be a temperature from the melting point to the boiling point of each of the reactants.

After the coupling reaction, the reaction mixture is added to a solution of a polymeric compound to cause their reaction. If the polymeric compound has a free amino group, for example, an acid amide bond is formed between the amino group and the carboxyl group of the hapten compound. The reaction temperature is 0°C to 60°C, preferably 5°C to 40°C, more preferably 22°C to 27°C. The reaction time is 5 minutes to 24 hours, preferably 1 to 16 hours, more preferably 1 to 2 hours. The reaction product is purified by dialysis and a desalting column, whereby the binding product between the antigen hapten and the polymeric compound can be obtained.

A mixed acid anhydride used in the mixed acid anhydride method is obtained by the reaction between a carboxylic acid and a haloformic acid ester. The resulting mixed acid anhydride is reacted with a polymeric compound to produce the desired hapten-polymeric compound binding product. This reaction is performed in the presence of a basic compound. Examples of the basic compound are organic bases such as tributylamine, triethylamine, trimethylamine, N-methylformalin, pyridine, N,N-dimethylaniline, DBN, DBU, and DABCO, and inorganic bases such as potassium carbonate, sodium carbonate, potassium hydrogencarbonate, and sodium hydrogencarbonate. The reaction is performed usually at -20°C to 100°C, preferably at 0°C to 50°C. The reaction time is 5 minutes to 10 hours, preferably 5 minutes to 2 hours. The reaction between the resulting mixed acid anhydride and the polymeric compound is performed usually at -20°C to 150°C, preferably at 0°C to 100°C. The reaction time is 5 minutes to 10 hours, preferably 5 minutes to 5 hours. The mixed acid anhydride method is performed generally in a solvent. The solvent may be any solvent in customary use for the mixed acid anhydride method. Examples of the solvent are ethers such as dioxane, diethyl ether, tetrahydrofuran, and dimethoxyethane, halogenated hydrocarbons such as dichloromethane, chloroform, and dichloroethane, aromatic hydrocarbons such as benzene, toluene and xylene, esters such as methyl acetate and ethyl acetate, and aprotic polar solvents such as N,N-dimethyl-formamide, dimethyl sulfoxide, and hexamethylphosphoric triamide. Examples of the haloformic acid ester used in the mixed acid anhydride method are methyl chloroformate, methyl bromoformate, ethyl chloroformate, ethyl bromoformate, and isobutyl chloroformate. The ratio of the hapten, haloformate, and polymeric compound used in this method can be suitably selected from a broad range.

In the same method as described above, the antigen hapten conjugated to a labeling substance such as an enzyme can be used in detection of the antigen. Examples of the labeling substance are enzymes such as horseradish peroxidase (hereinafter, "HRP"), and alkaline phosphatase, fluorescent substances such as fluorescein isocyanate and Rhodamine, radioactive substances such as ³²P and ¹²⁵I, and chemiluminescent substances.

### Preparation of polyclonal antibody

Using the binding product between the antigen hapten and the polymeric compound, a polyclonal antibody can be prepared by the customary method. For example, antigen hapten-BSA binding product is dissolved in sodium phosphate buffer (hereinafter, "PBS"), and mixed with an adjuvant such as complete or incomplete Freund's adjuvant, or alum. The mixture is administered as an immunizing antigen to an animal, whereby the polyclonal antibody can be obtained. The animal to be immunized may be any animal which is usually used in the field of the art. For example, it may be mouse, rat, rabbit, goat, or horse.

The method of administration for immunization may be subcutaneous injection, intraperitoneal injection, intravenous injection, intradermal injection, or intramuscular injection, but subcutaneous injection or intraperitoneal injection is preferred. Immunization can be performed once, or a plurality of times at suitable intervals, preferably at intervals of 1 to 5 weeks.

Blood is taken from the immunized animal, and serum is separated. Using the serum, the presence of a polyclonal antibody reactive with the antigen can be evaluated.

Antiserum, which has been obtained by using the binding product of the antigen hapten and the polymeric compound as an immunizing antigen, can react with an antigen by the indirect competitive inhibition ELISA method, for example, at a concentration of about 10 ng/ml.

### Preparation of monoclonal antibody

Using a binding product of an antigen hapten and a polymeric compound, a monoclonal antibody can be prepared by a publicly known method.

In preparing the monoclonal antibody, at least the following steps are required:
(a) Preparation of a binding product between an antigen hapten and a polymeric compound for use as an immunizing antigen.
(b) Immunization of an animal
(c) Sampling of blood, assay, and preparation of an antibody-forming cell
(d) Preparation of a myeloma cell
(e) Cell fusion of the antibody-forming cell and the myeloma cell, and selective culturing of hybridomas
(f) Screening for the hybridoma producing the desired antibody, and cell cloning
(g) Preparation of a monoclonal antibody by culturing of the hybridoma, or transplantation of the hybridoma to an animal
(h) Measurement of reactivity of the resulting monoclonal antibody

The usual method for preparing a hybridoma which produces a monoclonal antibody is described, for example, in Hybridoma Techniques (Cold Spring Harbor Laboratory, 1980), or Cell Histochemistry (Shuji Yamashita et al., edited by the Japan Society of Tissue Cytochemistry; Gakusai Kikaku, 1986).

The method for preparing a monoclonal antibody against the antigen will be described below, but not restricted to the following method, as will be clear among people skilled in the art:

The steps (a) to (b) can be performed by nearly the same methods as have been described in connection with the polyclonal antibody.

The antibody-forming cell in the step (c) is a lymphocyte, which can generally be obtained from the spleen, thymus, lymph node, peripheral blood, or a combination of these, but a spleen cell is used most generally. Therefore, after final immunization, a site where an antibody-forming cell exists, e.g., spleen, is removed from the mouse confirmed to form an antibody. From the spleen, spleen cells are prepared.

Examples of the myeloma cell usable in the step (d) are myeloma strain cells such as Balb/c mouse-derived myeloma cell strains P3/X63-Ag8 (X63) (Nature, 256, 495-497 (1975)), P3/X63-Ag8.U1 (P3U1) (Current Topics. in Microbiology and Immunology, 81, 1-7 (1987)), P3/NSI-1-Ag 4-1 (NS-1) (Eur. J. Immunol., 6, 511-519 (1976)), Sp2/O-Ag14 (Sp2/O) (Nature, 276, 269-270 (1978)), FO (J. Immuno. Meth., 35, 1-21 (1980)), MPC-11, X63.653, and S194; and rat-derived 210.RCY3.Ag1.2.3 (Y3) (Nature, 277, 131-133 (1979)).

The above-mentioned strain cell is subcultured in a Dulbecco modified Eagle's medium (DMEM) or Iscoff modified Dulbecco medium (IMDM) containing bovine fetal serum, and about 1x10⁶ cells or more are secured on the day of cell fusion.

The cell fusion in the step (e) can be performed in accordance with a publicly known method, e.g., the method of Milstein et al. (Methods in Enzymology, 73, 3 (1981)). The currently most generally used one is a method using polyethylene glycol (PEG). The PEG method is described, for example, in Cell Histochemistry, Yamashita Shuji et al. (already mentioned). Another method of fusion may be one by electric treatment (electrofusion) (Okouchi Etsuko et al., Experimental Medicine 5., 1315-19, 1987). Other method may be used where necessary. The ratio of the cells used may be the same as in the publicly known method. For example, spleen cells may be used in an amount of 3 to 10 times the number of the myeloma cells.

The selection of hybridomas gaining antibody-secreting ability and proliferating ability, which have been produced by fusion of the spleen cells and myeloma cells, can be done, for example, by the use of an HAT medium prepared by adding hypoxanthine-aminopterin-thymidine to the aforementioned DMEM or IMDM, if a hypoxanthine-guanine phosphoribosyltransferase deficient strain, for example, is used as the myeloma cell strain.

In the step (f), part of the culture supernatant containing the hybridoma group selected is taken, and measured for the antibody activity against the antigen, for example, by ELISA to be described later on.

Further, the hybridoma that has been found by the measurement to form an antibody reactive with the antigen is subjected to cell cloning. Methods for cell cloning include, for example, "limiting dilution analysis" for diluting the sample by limiting dilution such that one hybridoma is contained in one well; a method of seeding the sample on a soft agar medium, and recovering colonies; a method of withdrawing a single cell by a micromanipulator; and "sorter clone method" for separating a single cell by a cell sorter. The limiting dilution method is simple, and used often.

For the wells showing the antibody titer, cloning is repeated once to four times, for example, by limiting dilution, and the sample showing the antibody titer stably is selected as an anti-antigen monoclonal antibody-forming hybridoma strain. As a culture medium for culturing the hybridoma, DMEM or IMDM containing bovine fetal serum (FCS), for example, is used. Culturing of the hybridoma is preferably performed, for example, at a carbon dioxide concentration of about 5 to 7% and at 37°C (in an incubator at humidity of 100%).

Large scale culture for preparing the antibody in the step (g) is performed by, say, a follow fiber type culture device. Alternatively, it is possible to proliferate the hybridoma intraperitoneally in the mouse of the same strain (e.g., the aforementioned Balb/c) or Nu/Nu mouse, and prepare the antibody from ascitic fluid.

The culture supernatant or ascitic fluid obtained through the above step can be used as an anti-antigen monoclonal antibody, but may be further subjected to dialysis, salting out by ammonium sulfate, gel filtration, or lyophilization. Antibody fractions are collected and purified, whereby the anti-antigen monoclonal antibody can be obtained. If further purification is necessary, this can be achieved by combining methods in customary use, such as ion exchange column chromatography, affinity chromatography, and high performance liquid chromatography (HPLC).

From the monoclonal antibody against the antigen that has been obtained in the above manner, subclasses and antibody titer can be determined by known methods such as ELISA.

In FIGS. 3 and 4, the antibody 19 can form a covalent bond to the fixing layer 16 by a publicly known method. For example, there can be employed the aforementioned activated ester method (A.E. KARU et al.: J. Agric. Food Chem. 42 301-309 (1994)), or the aforementioned mixed acid anhydride method (B.F. Erlanger et al.: J. Biol. Chem. 234 1090-1094 (1954)), and other publicly known coupling methods.

Publicly known coupling methods are shown in FIGS. 13 and 14. The coupling methods of FIGS. 13 and 14 are described in detail in V.P. Torchilin:Adv. Drug Delivery Rev., 1, 41 (1987), and the Chemical Society of Japan, "4th Ed., Lecture on Experimental Chemistry 29, Polymeric Materials", Maruzen Co., Ltd., September 25, 1993.

Typically, a sensor chip to which the antibody 19 has not been fixed is embedded into a predetermined position of a predetermined holder which can be inserted into an SPR apparatus obtainable from BIACORE (registered trademark) Co., Ltd. Then, the holder is inserted into the SPR apparatus, and a predetermined reaction mix is flowed into the SPR apparatus to fix the antibody 19 onto the surface of the sensor chip.

FIGS. 5A and 5B show the SPR apparatus, especially, its flow cell structure, and the sensor chip of the invention for surface plasmon resonance. In FIG. 5A, an optical system such as a prism 34 and an optical interface 38, a sensor chip 10, and a microflow passage system such as a cell structure 42 are shown in a separated state for convenience of explanation. FIG. 5B, on the other hand, is a sectional view of the optical system, sensor chip 10, and microflow passage system.

In FIGS. 5A and 5B, the numeral 10 denotes the sensor chip of the invention for surface plasmon resonance. Its example is the sensor chip for surface plasmon resonance shown in FIGS. 3 and 4. Typically, the sensor chip 10 is formed in a holder, and the holder is inserted into the SPR apparatus, whereby the sensor chip 10 is fixed between the optical system and the microflow passage system. This holder can be withdrawn from the SPR apparatus.

On one side of the sensor chip 10, the microflow passage system is disposed. On the other side of the sensor chip 10, the optical system is disposed.

The optical system has a light source (not shown), the prism 34, the optical interface 38 between the prism and the sensor chip 10, and a detector (not shown). As the light source, a light emitting diode is preferably used. For example, polarized light with a wavelength of 760 nm is condensed by the prism 34 into wedge-shaped light, which is projected onto the sensor chip 10 under total reflection conditions. The intensity of wedge-shaped reflected light reflected by the sensor chip 10 is monitored with a detector such as a fixed diode array. The intensity is analyzed by a computer to calculate the SPR angle automatically.

The microflow passage system has a substrate 40, and the cell structure 42 having a recess 42 formed in the surface. The recess 42 has wall surfaces on three sides, and is open upwards. The sensor chip 10 can be mounted so as to cover the cell structure 42 from above, whereby a flow cell 46 can be formed. Preferably, there are two or more of the flow cells 46, and four flow cells are formed in FIGS. 5A and 5B.

In correspondence with the four flow cells 46, four of the sensor chips 10 are formed. That is, in correspondence with each of the four flow cells 46 shown in FIG. 5B, the metallic thin film 14 and the fixing layer 16 are formed on the surface of the substrate 12 shown in FIGS. 3 and 4. On the other hand, on the surface of the substrate 12 corresponding to the portions where no flow cells are arranged in FIG. 5B, e.g., the portions between the flow cells, it is not necessary for the metallic thin film 14 and the fixing layer 16 to be formed.

In the flow cell, an antigen which has flowed through the microflow passage system is bound to the antibody 19 fixed to the surface of the sensor chip 10.

FIG. 6A shows the microflow passage system of the SPR apparatus, and FIG. 6B is a partial enlarged sectional view of FIG. 6A.

A microflow passage system 50 has a sample injection port 52 and a buffer injection port 54. Preferably, a sample and a buffer can be injected by a syringe pump, and can be fed at a constant flow rate in the range of 1 to 100 µl/min.

A sample placed on a sample rack (not shown) is automatically mixed and diluted by an autosampler (not shown), and injected through the sample injection port 52. The injected solution is transported to a flow cell 46 through the microflow passage system, and brought into contact with the surface of the sensor chip 10 at the flow cell 46.

The height of a sample loop in the microflow passage system is, for example, 50 µm. Between the sample loop and the flow cell, a plurality of tiny diaphragm valves 56, which act under compressed air, are built in to control feed of the solution to the sample loop and the flow cell in the microflow passage system. Opening and closing of these valves are automatically controlled by computer software in accord with the injection of the sample.

FIG. 7 shows the flow cell portion of the microflow passage system. In FIG. 7, valves 48b, 48c, 49c are opened, while valves 49a, 49b, 48d are closed. Thus, the sample flows through flow cells 46a, 46b, 46c in this order.

For example, anti-estradiol-17β antibody may be fixed to the sensor chip corresponding to the flow cell 46a, anti-dioxin antibody may be fixed to the sensor chip corresponding to the flow cell 46b, and anti-PCB antibody may be fixed to the sensor chip corresponding to the flow cell 46c. By merely introducing the sample once into the SPR apparatus, estradiol-17β, dioxin and PCB in the sample can be detected.

If the sensor chip has two or more antibodies against two or more antigens as stated above, the two or more antigens can be detected by performing the procedure once. This is efficient.

By operating the valves, several modes can be selected, such as the mode of flowing the sample into one flow cell, or the mode of flowing the sample through the flow cells sequentially. In the microflow passage system, air valves partition the sample and the buffer. Thus, the dilution and diffusion of the sample can be minimized, and the duration of contact between the sample and the sensor chip 10 can be controlled accurately.

FIGS. 8A, 8B and 8C are explanation drawings of the competitive method. With plasmon resonance analysis, a change due to binding of an antigen 29 to an antibody 19 fixed to a sensor chip 10 is detected as a change in disappearance angle.

When the molecular weight of the antigen 29 is small, however, the change in the disappearance angle also becomes small, and so may be difficult to detect. Hence, when the molecular weight of the antigen 29 is small, the disappearance angle can be detected by the competitive method described below.

With the competitive method, a labeled antigen, i.e., an antigen bound to a suitable polymeric compound, is used. The labeled antigen is obtained typically by coupling the antigen to the polymeric compound. The method of coupling may be the same method as used to bind an antigen hapten to a suitable polymeric compound in preparing an antibody. For example, the coupling methods shown in FIGS. 13 and 14 can be used. As the polymeric compound, the same one as shown there may be used.

Then, the labeled antigen, and one or more mixtures of the unlabeled antigen and the labeled antigen mixed in predetermined proportions are measured for the disappearance angle with the use of the sensor chip for surface plasmon resonance.

In FIG. 8A, a standard sample containing a labeled antigen 29a is flowed through the flow passage, whereby the labeled antigen 29a is bound to the antibody 19 of the sensor chip 10.

In FIG. 8B, a mixture of the above standard sample and a sample containing an antigen 29 mixed in predetermined proportions is flowed through the flow passage.
Proportional to the ratio between the labeled antigen 29a and the antigen 29 in the mixture, the labeled antigen 29a and the antigen 29 are bound to the antibody 19 of the sensor chip 10.

In FIG. 8C, a mixture of the above standard sample and a sample containing an antigen 29 mixed in predetermined proportions is flowed through the flow passage, as in FIG. 8B. In FIG. 8C, however, the proportion of the sample containing the antigen 29 is higher than in FIG. 8B.

That is, in the order of FIGS. 8A, 8B and 8C, the concentration of the labeled antigen 29 decreases, while the concentration of the antigen 29 increases. Since the labeled antigen 29a has a large molecular weight, this decrease in the concentration of the labeled antigen 29a can be detected as a change in the disappearance angle.

### Examples

Dioxin in a sample was measured by the use of an SPR apparatus, called BIAcore (registered trademark) 2000, which is produced by BIACORE (Upsula, Sweden) and can be obtained from Biacore Kabushiki Kaisha (3-25-1, Hyakunin-cho, Shinjuku-ku, Tokyo, Japan).

### Example 1

An anti-dioxin monoclonal antibody was fixed to a sensor chip by the aminocoupling method using BIAcore (registered trademark) 2000.

As a sensor chip to which the antibody was not fixed, a sensor chip (commercial name CM5) available from BIAcore company was used. This sensor chip has a structure as shown in FIG. 3. The metallic thin film 14 is composed of gold, and its thickness is 50 nm. The fixing body 18 is carboxymethyldextran having no crosslinked structure, and its thickness is 100 nm.

As the anti-dioxin monoclonal antibody, Catalog No. PDI-Dioxin-30 (Research Diagnostics, Inc., Pleasant Hill Road, Flanders, New Jersey, USA) was used. As the host, mouse was used. This anti-dioxin monoclonal antibody contains 10 mg IgG1 in 3.125 ml (3.2 mg/ml), 0.2M PBS, pH 7.2, and 0.05% sodium azide.

The reactivity of the anti-dioxin monoclonal antibody is as follows:

| | |
|---|---|
| 2,3,7,8-TCDD | 100% |
| 2,3,7,8-TCDF | 4.6% |
| 2,3,7-TriCDD | <20% |
| 2,3-DCDD | 2.2% |
| 2-CDD | 0.3% |
| 1,2,4-TriCDD | <1.0% |

First, BIAcore's sensor chip CM5 was set in BIAcore (registered trademark) 2000. Then, a mixed solution of N-ethyl-N'-(3-dimethylaminopropyl)carbodiimide hydrochloride (EDC) and N-hydroxysuccinimide (NHS) was injected into BIAcore (registered trademark) 2000 to activate the sensor chip CM5.

The anti-dioxin monoclonal antibody (1,000 ppm) was centrifuged to replace the solvent. Finally, the antibody was dissolved in 100 µl of 10 mM acetic acid environment solution. The resulting solution in an amount of 50 µl was injected into BIAcore (registered trademark) 2000 at a rate of 5 µl/min to fix the anti-dioxin monoclonal antibody onto the surface of the sensor chip.

Ethanolamine was injected for blocking of the unreacted active site on the surface of the sensor chip. Finally, 0.1N hydrochloric acid was flowed for washing.

### Example 2

Using the sensor chip obtained in Example 1, dioxins were detected by the competitive method. In Example 2, the temperature inside the apparatus was kept at 25°C.

Inside the BIAcore 2000, 7.6 ppm of a 2,3,7,8-TCDD/methanol solution was diluted with a running buffer to 76 to 3.8 ppb. The running buffer (HBS) was an aqueous solution containing 0.01M HEPES, 0.15M NaCl, 3 mM EDTA, and 0.005% Polysorbate 20 (v/v).

The diluted 2,3,7,8-TCDD solution of each concentration was mixed with 10 ppm HRP-2,3,7,8-TCDD in BIAcore. The HRP-2,3,7,8-TCDD refers to HRP-2,3,7,8-TCDD labeled with horse radish peroxidase (HRP) by aminocoupling. A change in the disappearance angle due to the binding of 2,3,7,8-TCDD itself to the antibody is too small to be measured by SPR. Thus, the labeled 2,3,7,8-TCDD was used.

Then, the mixture was injected (3 µl/min) into BIAcore 2000, and an SPR signal was measured to determined the disappearance angle. That is, polarized light with a wavelength of 760 nm was projected, and the intensity of reflected light was measured, with the angle of incidence of incident light being varied. Between respective measurements, the system was washed with 0.1N hydrochloric acid.

The results are shown in Table 4 and FIG. 9.

**Table 4**

| Dioxin concentration | 3.8 ppb | 7.6ppb | 76 ppb |
|---|---|---|---|
| ΔRU | 256.5 | 230.6 | 170.6 |
| | 273.7 | 230.3 | 152.4 |
| | 272.3 | 223.5 | 146.3 |
| | 136.5 | 107.2 | 74.3 |
| Average | 234.75 | 197.9 | 135.9 |

The data obtained from the four cells were averaged to prepare a graph as shown in FIG. 9. The decrease in the binding of HRP-2,3,7,8-TCDD indicates that 2,3,7,8-TCDD bound to the antibody.

1,000 RU corresponds to a disappearance angle of 0.1 degree. Usually, the change in the disappearance angle is small with SPR measurement, so that resonance unit (RU) is used.

FIG. 10 shows the correlation between the elapsed time after fixing of the anti-dioxin monoclonal antibody and the response value.

### Examples 3

An anti-estradiol antibody was fixed to a sensor chip by the aminocoupling method using BIAcore (registered trademark) 2000. Operation of the interior of the BIAcore (registered trademark) 2000 was performed at 25°C.

As a sensor chip to which the antibody was not fixed, a sensor chip (commercial name CM5) available from BIAcore company was used. This sensor chip has the structure shown in FIG. 3. The metallic thin film 14 is composed of gold, and its thickness is 50 nm. The fixing body 18 is carboxymethyldextran having no crosslinked structure, and its thickness is 100 nm.

As the anti-estradiol antibody, estradiol antiserum FKA204 obtained from Cosmobio Kabushiki Kaisha (Toyo 2-2-20, Koto-ku, Tokyo 135-0016, Japan) was used. This antigen is 6-oxo-estradiol-6-CMO-BSA IgG.

As a running buffer, BIAcore's HBS buffer was used. This running buffer contains 10 mM HEPES, 15 mM NaCl, 3 mM EDTA, and 0.005% v/v Surfactant P20, pH 7.4.

First, BIAcore's sensor chip CM5 was set in BIAcore (registered trademark) 2000. Then, a mixed solution of N-ethyl-N'-(3-dimethylaminopropyl)carbodiimide hydrochloride (EDC) and N-hydroxysuccinimide (NHS) was injected (5 µl/min) into BIAcore (registered trademark) 2000 to activate the sensor chip CM5.

The estradiol antiserum (Cosmobio) was diluted 1:50 with 10 mM acetate buffer (pH 4.8) The amount of the final solution was 100 µl. The resulting solution in an amount of 50 µl was injected into BIAcore (registered trademark) 2000 at a rate of 5 µl/min to fix the anti-estradiol monoclonal antibody onto the surface of the sensor chip.

40 µL of 1M ethanolamine (pH 8.5) was injected for blocking of the unreacted active site on the surface of the sensor chip. Finally, 5 µL of 0.1N hydrochloric acid was flowed for washing.

### Example 4

Using the sensor chip obtained in Example 3, estradiol was detected by the competitive method. In Example 4, the temperature inside the apparatus was kept at 25°C. As a running buffer, 50 mM of phosphate buffer (pH 7.5) was used.

A plurality of estradiol solutions adjusted to predetermined concentrations were used. One part by volume of each estradiol solution and 4 parts by volume of 10 ppm HRP-labeled estradiol were mixed inside BIAcore 2000. The HRP-labeled estradiol refers to estradiol labeled with horse radish peroxidase (HRP) by aminocoupling.

Then, 35 µl of each mixture was injected (5 µl/min) into BIAcore 2000, and an SPR signal was measured to determined the disappearance angle. That is, polarized light with a wavelength of 760 nm was projected, and the intensity of reflected light was measured, with the angle of incidence of incident light being varied.

Further, 50 µL of 100 ppm anti-HRP antibody was injected (5 µl/min) into BIAcore, and the disappearance angle was measured. In this manner, the competitive method and the sandwich method can be used jointly.

Between respective measurements, the system was washed with 0.1N hydrochloric acid.

The buffer for 50 ppm HRP-labeled estradiol (Cosmobio) was replaced by use of Molcut (Millipore; fractional molecular weight 10,000).

The buffer for 1,000 ppm anti-HRP antibody (Biomeda) was replaced by use of Molcut (Millipore; fractional molecular weight 10,000) to dilute the antibody to 100 ppm.

The results are shown in Table 5 and FIG. 11. The data obtained from the four cells were averaged to prepare a graph as shown in FIG. 11.

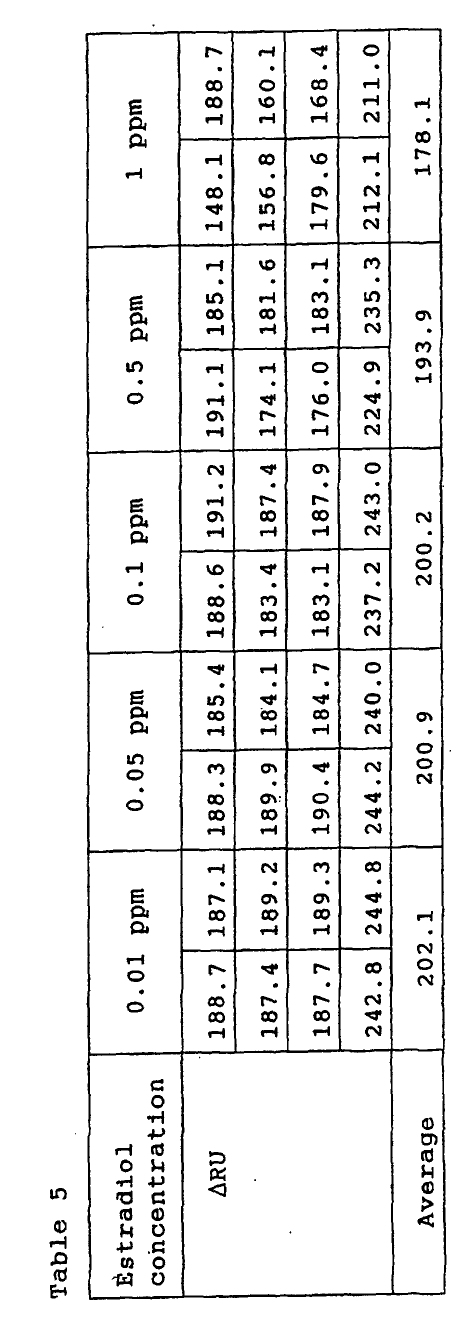

The present invention can detect an antigen with high sensitivity by utilizing surface plasmon resonance. Its sensitivity may reach, for example, about 0.1 ppb. The invention can also detect the antigen qualitatively and quantitatively. Furthermore, the invention is simple in procedure in comparison with the conventional GC-MS method, or methods using enzyme reactions.

Besides, the invention uses an antigen-antibody reaction, and thus is excellent in selectivity. According to the invention, it is possible to detect a substance which binds to an antibody against a substance having a steroid skeleton, a steroid hormone, a sex hormone, or an endocrine disruptor. Since such a substance may be a new endocrine disruptor, the invention is useful for screening of endocrine disruptors.

The invention is not restricted to the detection of substances having a steroid skeleton, steroid hormones, sex hormones, or endocrine disruptors, or to screening of endocrine disruptors. The invention can be applied to various tests, such as test for abnormal secretion of sex hormone (e.g., test for sterility), test for pregnancy, test for menopausal syndrome, and doping test in athletes.

## Claims

1. A method for detecting an antigen, comprising the steps of:
exposing an antibody of a sensor chip for surface plasmon resonance to a sample while said sample being in liquid is flowed through a microflow passage system, said sensor chip having a metallic thin film; one or more of the antibodies against one or more antigens selected from a substance having a steroid skeleton, a substance for maintaining, promoting or inhibiting the physiological action of a steroid hormone a substance for maintaining, promoting or inhibiting the physiological action of a sex hormone, and an endocrine disruptor (excluding a triazine compound); and a fixing layer for fixing the antibody to the metallic thin film;
irradiating the metallic thin film with light; and detecting intensity of reflected light from the metallic thin film.

2. The method for detecting an antigen as claimed in claim 1, wherein said microflow passage system comprises one or more flow cells in which the liquid sample is flown and exposed to the antibody fixed on the sensor chip.

3. The method for detecting an antigen as claimed in claim 1 or 2, further including the step of finding a shift in a disappearance angle of the reflected light.

4. The method for detecting an antigen as claimed in claim 1, 2, or 3, further including the step of binding a labelled antigen, which is the antigen bound to a labelling material, to the antibody before the exposing step.

5. The method for detecting an antigen as claimed in claim 1, 2, 3 or 4, wherein the antibody is an antibody against a steroid hormone, or against a substance showing the same physiological action as that of a steroid hormone.

6. The method for detecting an antigen as claimed in claim 1, 2, 3 or 4, wherein the antibody is an antibody against a sex hormone, or against a substance showing the same physiological action as that of a sex hormone.

7. The method for detecting an antigen as claimed in claim 1, 2, 3 or 4, wherein the antibody is an antibody against an estrogen.

8. The method for detecting an antigen as claimed in claim 1, 2, 3 or 4, wherein the antibody is an antibody against an endocrine disruptor having a cyclic hydrocarbon portion.

9. The method for detecting an antigen as claimed in claim 1, 2, 3 or 4, wherein the antibody is an antibody against dioxins.

10. The method for detecting an antigen as claimed in any one of claims 1-9, wherein the sensor chip for surface plasmon resonance has two or more of the antibodies against two or more of the antigens, and can detect the two or more antigens.

11. A surface plasmon resonance apparatus comprising:
a sensor chip for surface plasmon resonance, having a metallic thin film,
one or more antibodies against one or more antigens selected from a substance having a steroid skeleton, a substance for maintaining, promoting or inhibiting the physiological action of a steroid hormone, a substance for maintaining, promoting or inhibiting the physiological action of a sex hormone, and an endocrine disruptor (excluding a triazine compound), and
a fixing layer for fixing the antibody to the metallic thin film;
an optical system having
a light source for irradiating the sensor chip for surface plasmon resonance with irradiation light,
a detector for detecting reflected light from the sensor chip for surface plasmon resonance, and
a prism pervious to at least the irradiation light; and
a microflow passage system for flowing a liquid sample therein to bring the sample into contact with the antibody of the sensor chip for surface plasmon resonance.

12. The surface plasmon resonance apparatus of claim 11, wherein the antibody is an antibody against a steroid hormone, or against a substance showing the same physiological action as that of a steroid hormone.

13. The surface plasmon resonance apparatus of claim 11, wherein the antibody is an antibody against a sex hormone, or against a substance showing the same physiological action as that of a sex hormone.

14. The surface plasmon resonance apparatus of claim 11, wherein the antibody is an antibody against an estrogen.

15. The surface plasmon resonance apparatus of claim 11, wherein the antibody is an antibody against an endocrine disruptor having a cyclic hydrocarbon portion.

16. The surface plasmon resonance apparatus of claim 11, wherein the antibody is an antibody against dioxins.

17. The surface plasmon resonance apparatus of any one of claims 11-16, wherein the microflow passage system has one or more flow cells, and the sensor chip for surface plasmon resonance is located at a position corresponding to said one or more flow cells.

## Patentansprüche

1. Verfahren zum Nachweisen eines Antigens, umfassend die Schritte:
des Aussetzens eines Antikörpers auf einen Sensorchip für die Oberflächenplasmonresonanz gegenüber einer Probe, während diese Probe, die in einer Flüssigkeit ist, durch ein Mikroflußpassagensystem fließen gelassen wird, wobei der Sensorchip einen Metallfilm hat und einen oder mehrere der Antikörper gegen ein oder mehrere Antigene, die aus einer Substanz, die ein Steroidskelett hat, einer Substanz zum Bewahren, Unterstützen oder Inhibieren der physiologischen Wirkung eines Steroidhormons, einer Substanz zum Aufrechterhalten, Fördern und Inhibieren der physiologischen Wirkung eines Sexualhormons, und eines endokrinen Unterbrechers (ausgenommen eine Triazinverbindung) ausgewählt sind, und eine Fixierungsschicht zum Fixieren des Antikörpers auf der dünnen Metallschicht;
des Bestrahlens der dünnen Metallschicht mit Licht und Nachweisen der Intensität des von der dünnen Metallschicht reflektierten Lichts.

2. Verfahren zum Nachweisen eines Antigens wie in Anspruch 1 beansprucht, wobei das Mikroflußpassagensystem eine oder mehrere Fließzellen umfaßt, durch die die Flüssigprobe geleitet wird und dem Antikörper ausgesetzt wird, der auf dem Sensorchip fixiert ist.

3. Verfahren zum Nachweisen eines Antigens wie in Anspruch 1 oder 2 beansprucht, welches außerdem den Schritt des Auffindens eines Wechsels eines Austrittswinkels des reflektierten Lichts einschließt.

4. Verfahren zum Nachweisen eines Antigens wie in Anspruch 1, 2 oder 3 beansprucht, welches den Schritt des Bindens eines markierten Antigens an einen Antikörper vor dem Expositionsschritt einschließt, wobei das Antigen an das Markierungsmaterial gebunden ist.

5. Verfahren zum Nachweisen eines Antigens wie in Anspruch 1, 2, 3 oder 4 beansprucht, wobei der Antikörper ein Antikörper gegen ein Steroidhormon ist oder gegen eine Substanz, die die gleiche physiologische Wirkung wie die eines Steroidhormons zeigt.

6. Verfahren zum Nachweisen eines Antigens wie in Anspruch 1, 2, 3 oder 4 beansprucht, wobei der Antikörper ein Antikörper gegen ein Sexualhormon ist oder gegen eine Substanz, die die gleiche physiologische Wirkung wie die eines Sexualhormons zeigt.

7. Verfahren zum Nachweisen eines Antigens wie in Anspruch 1, 2, 3 oder 4 beansprucht, wobei der Antikörper ein Antikörper gegen ein Östrogen ist.

8. Verfahren zum Nachweisen eines Antigens wie in Anspruch 1, 2, 3 oder 4 beansprucht, wobei der Antikörper ein Antikörper gegen einen endokrinen Unterbrecher mit einem zyklischen Kohlenwasserstoffanteil ist.

9. Verfahren zum Nachweisen eines Antigens wie in Anspruch 1, 2, 3 oder 4 beansprucht, wobei der Antikörper ein Antikörper gegen Dioxine ist.

10. Verfahren zum Nachweisen eines Antigens wie in jedem der Ansprüche 1 bis 9 beansprucht, wobei der Sensorchip für die Oberflächenplasmonresonanz zwei oder mehr der Antikörper gegen zwei oder mehr der Antigene hat und die zwei oder mehr Antigene nachweisen kann.

11. Oberflächenplasmonresonanz-Apparat, umfassend:
einen Sensorchip für die Oberflächenplasmonresonanz mit einer dünnen Metallschicht,
einen oder mehrere Antikörper gegen ein oder mehrere Antigene ausgewählt aus einer Substanz mit einem Steroidskelett, einer Substanz zum Aufrechterhalten, Fördern oder Inhibieren der physiologischen Wirkung eines Steroidhormons, einer Substanz zum Aufrechterhalten, Fördern oder Inhibieren der physiologischen Wirkung eines Sexualhormons, und einen endokrinen Unterbrecher (ausgenommen eine Triazinverbindung) und
einer Fixierungsschicht zum Fixieren des Antikörpers auf der dünnen Metallschicht;
ein optisches System mit
einer Lichtquelle zum Bestrahlen des Sensorchips für die Oberflächenplasmonresonanz mit Bestrahlungslicht,
einem Detektor zum Nachweisen des reflektierten Lichts von dem Sensorchip für die Oberflächenplasmonresonanz und
einem Prisma, das mindestens gegenüber dem Bestrahlungslicht durchlässig ist; und
ein Mikroflußpassagensystem zum Durchleiten einer Flüssigprobe darin, um die Probe mit dem Antikörper des Sensorchips für die Oberflächenplasmonresonanz in Kontakt zu bringen.

12. Oberflächenplasmonresonanz-Apparat nach Anspruch 11, wobei der Antikörper ein Antikörper gegen ein Steroidhormon oder gegen eine Substanz mit der gleichen physiologischen Wirkung wie die eines Steroidhormons ist.

13. Oberflächenplasmonresonanz-Apparat nach Anspruch 11, wobei der Antikörper ein Antikörper gegen ein Sexualhormon oder gegen eine Substanz mit der gleichen physiologischen Wirkung wie die eines Sexualhormons ist.

14. Oberflächenplasmonresonanz-Apparat nach Anspruch 11, wobei der Antikörper ein Antikörper gegen ein Östrogen ist.

15. Oberflächenplasmonresonanz-Apparat nach Anspruch 11, wobei der Antikörper ein Antikörper gegen einen endokrinen Unterbrecher mit einem zyklischen Kohlenwasserstoffteil ist.

16. Oberflächenplasmonresonanz-Apparat nach Anspruch 11, wobei der Antikörper ein Antikörper gegen ein Dioxine ist.

17. Oberflächenplasmonresonanz-Apparat nach jedem der Ansprüche 11 bis 16, wobei das Mikroflußpassagensystem eine oder mehrere Fließzellen hat und der Sensorchip für die Oberflächenplasmonresonanz in einer Position lokalisiert ist, die einer oder mehreren Fließzellen entspricht.

## Revendications

1. Procédé pour détecter un antigène, comprenant les étapes de :
l'exposition d'un anticorps d'une puce capteur pour résonance plasmon de surface à un échantillon pendant que ledit échantillon qui est sous forme liquide s'écoule à travers un système de passage de micro-écoulement, ladite puce capteur ayant un film métallique mince ; un ou plusieurs des anticorps contre un ou plusieurs antigènes choisis parmi une substance ayant un squelette stéroïde, une substance pour maintenir, promouvoir ou inhiber l'action physiologique d'une hormone stéroïde, une substance pour maintenir, promouvoir ou inhiber l'action physiologique d'une hormone sexuelle, et un disrupteur endocrinien (excepté un composé triazine) ; et une couche de fixation pour fixer l'anticorps au film métallique mince ;
l'irradiation du film métallique mince avec de la lumière ; et la détection de l'intensité de la lumière réfléchie par le film métallique mince.

2. Procédé pour détecter un antigène comme revendiqué dans la revendication 1, dans lequel ledit système de passage de micro-écoulement comprend une ou plusieurs cellules d'écoulement dans lesquelles l'échantillon liquide s'écoule et est exposé à l'anticorps fixé sur la puce capteur.

3. Procédé pour détecter un antigène comme revendiqué dans la revendication 1 ou 2, comprenant en outre l'étape de recherche d'un déplacement dans un angle de disparition de la lumière réfléchie.

4. Procédé pour détecter un antigène comme revendiqué dans la revendication 1, 2 ou 3, comprenant en outre l'étape de liaison d'un antigène marqué, qui est l'antigène lié à un matériau de marquage, à l'anticorps avant l'étape d'exposition.

5. Procédé pour détecter un antigène comme revendiqué dans la revendication 1, 2, 3 ou 4, dans lequel l'anticorps est un anticorps contre une hormone stéroïde, ou contre une substance montrant la même action physiologique que celle d'une hormone stéroïde.

6. Procédé pour détecter un antigène comme revendiqué dans la revendication 1, 2, 3 ou 4, dans lequel l'anticorps est un anticorps contre une hormone sexuelle, ou contre une substance montrant la même action physiologique que celle d'une hormone sexuelle.

7. Procédé pour détecter un antigène comme revendiqué dans la revendication 1, 2, 3 ou 4, dans lequel l'anticorps est un anticorps contre un oestrogène.

8. Procédé pour détecter un antigène comme revendiqué dans la revendication 1, 2, 3 ou 4, dans lequel l'anticorps est un anticorps contre un disrupteur endocrinien ayant une partie hydrocarbonée cyclique.

9. Procédé pour détecter un antigène comme revendiqué dans la revendication 1, 2, 3 ou 4, dans lequel l'anticorps est un anticorps contre les dioxines.

10. Procédé pour détecter un antigène comme revendiqué dans l'une quelconque des revendications 1 à 9, dans lequel la puce capteur pour résonance plasmon de surface a deux ou plusieurs des anticorps contre deux ou plusieurs des antigènes, et peut détecter les deux ou plusieurs antigènes.

11. Appareil à résonance plasmon de surface comprenant :
une puce capteur pour résonance plasmon de surface, ayant un film métallique mince,
un ou plusieurs anticorps contre un ou plusieurs antigènes choisis parmi une substance ayant un squelette stéroïde, une substance pour maintenir, promouvoir ou inhiber l'action physiologique d'une hormone stéroïde, une substance pour maintenir, promouvoir ou inhiber l'action physiologique d'une hormone sexuelle, et un disrupteur endocrinien (excepté un composé triazine) ; et
une couche de fixation pour fixer l'anticorps au film métallique mince ;
un système optique ayant
une source de lumière pour irradier la puce capteur pour résonance plasmon de surface avec la lumière d'irradiation,
un détecteur pour détecter la lumière réfléchie depuis la puce capteur pour résonance plasmon de surface, et
un prisme perméable à au moins la lumière d'irradiation ; et
un système de passage de micro-écoulement pour l'écoulement d'un échantillon liquide dans celui-ci pour amener l'échantillon en contact avec l'anticorps de la puce capteur pour résonance plasmon de surface.

12. Appareil à résonance plasmon de surface selon la revendication 11, dans lequel l'anticorps est un anticorps contre une hormone stéroïde, ou contre une substance montrant la même action physiologique que celle d'une hormone stéroïde.

13. Appareil à résonance plasmon de surface selon la revendication 11, dans lequel l'anticorps est un anticorps contre une hormone sexuelle, ou contre une substance montrant la même action physiologique que celle d'une hormone sexuelle.

14. Appareil à résonance plasmon de surface selon la revendication 11, dans lequel l'anticorps est un anticorps contre un oestrogène.

15. Appareil à résonance plasmon de surface selon la revendication 11, dans lequel l'anticorps est un anticorps contre un disrupteur endocrinien ayant une partie hydrocarbonée cyclique.

16. Appareil à résonance plasmon de surface selon la revendication 11, dans lequel l'anticorps est un anticorps contre les dioxines.

17. Appareil à résonance plasmon de surface selon l'une quelconque des revendications 11 à 16, dans lequel le système de passage de micro-écoulement a une ou plusieurs cellules d'écoulement, et la puce capteur pour la résonance plasmon de surface est placée à un endroit correspondant auxdites une ou plusieurs cellules d'écoulement.
